# DEMANDE DE BREVET EUROPEEN

(11) **EP 1 769 774 A1**
(43) Date de publication de la demande: **04.04.2007**
(21) Numéro de dépôt: 05109136.1
(22) Date de dépôt: 03.10.2005
(51) Int. Cl.: A61F 2/06

(54) **Endoprothèse radio-opaque**

(71) Demandeur: Frid, Noureddine, 1650 Beersel (BE)
(72) Inventeur: Frid, Noureddine, 1650 Beersel (BE)
(74) Mandataire: Van Straaten, Joop

(57) **Abrégé**

Une endoprothèse munie d'une armature en fils métalliques visible en imagerie médicale sous rayons X dans laquelle l'armature est formée d'au moins trois couches de fils les fils formant l'armature comprennent une âme en un matériau radio-opaque, l'interaction entre les âmes des fils des différentes couches améliorant la visibilité de la dite endoprothèse en imagerie médicale sous rayons X.

## Description

### Domaine de l'invention

L'invention se rapporte des endoprothèses munies d'une armature en fils métalliques tressés dont la visibilité est améliorée en imagerie médicale.

L'invention se rapporte aussi à une méthode pour la réalisation de telles endoprothèses.

### État de la technique

La technique consistant à placer des endoprothèses luminales et notamment des stents dans des vaisseaux sanguins atteints de thromboses ou sur lesquels se développent des anévrismes et actuellement bien connue et largement appliquée, notamment dans le domaine cardio-vasculaire.

Il subsiste cependant un problème important lors du placement et lors de vérifications ultérieures sur l'état des patients : en raison de leur finesse, ces endoprothèses sont extrêmement difficiles à repérer par endoscopie par rayons X. La seule partie qui apparaisse en imagerie est leur armature, dont la masse et l'épaisseur vont décroissantes avec le diamètre des vaisseaux traités. Le cas le plus frappant est celui des interventions dans le volume de la boite crânienne : les os du crâne constituent par eux-mêmes un obstacle important pour l'absorption du rayonnement. Les stents pour le traitement des anévrismes cérébraux se caractérisent par ailleurs par leur faible diamètre, en général de l'ordre de 3 à 5 mm. Les fils formant ces stents sont eux-mêmes extrêmement fins (de l'ordre de 40 à 50 µm), de façon à ce qu'on puisse introduire le stent via un cathéter dont le diamètre extérieur ne dépasse pas 3 unités French. Lors du largage d'un stent de cet ordre de dimensions sous contrôle par rayons X, le stent, du fait de la faible épaisseur des fil utilisés et de la perte de contraste due à l'épaisseur de la boîte crânienne, est pratiquement impossible à repérer, ce qui veut dire que l'opérateur, pour ces opérations très délicates, doit travailler pratiquement à l'aveuglette.

En pratique, on considère que la plupart des fils perdent leur radio-opacité pour une épaisseur de l'ordre de 0.1 mm. L'usage de métaux nobles (fils en platine, or ou tantale) permettrait de descendre pour ce qui est de la visibilité jusqu'à un diamètre de l'ordre de 25µm. Malheureusement, les propriétés mécaniques de ces métaux sont médiocres, si bien qu'il n'est pas possible de les utiliser seuls : il faut obligatoirement les associer à d'autres métaux plus résistants.
Pour pallier à ce problème, diverses tentatives ont été élaborées. US 6569194 propose par exemple d'utiliser un alliage incorporant un métal tel que du tungstène, ce qui altère évidemment les performances mécaniques de l'armature.
US 2003/0121148 et WO 2005/028014 envisagent, pour les stents formés à partir de tubes découpés au laser, la fixation, par soudure, de pastilles en matériaux fortement absorbant, tels que le platine, l'or ou le tantale qui fonctionnent donc comme des marqueurs. Cette méthode nécessite de recourir, lors de la fabrication, à un équipement très élaboré et coûteux. La fixation de ces pastilles doit éventuellement se faire en deux étapes, car il faut recourir à une couche intermédiaire dite d'amorçage afin d'obtenir une adhérence suffisante. En outre, l'excès de matière doit être éliminé par électropolissage. Un autre problème qui est lié à cette technique est l'apparition de phénomènes de corrosion galvanique entre le matériau soudé et le métal de l'armature dans le milieu sanguin, agissant comme un électrolyte. Le même problème apparaît EP-0894481, où l'on place comme marqueurs sur l'armature des morceaux de fils de métal radio-opaques.

### Résumé de l'invention

Un but de l'invention est de rendre l'armature d'une endoprothèse visible en imagerie médicale, sans utiliser des quantités importantes de métaux radio-opaques, dont le prix est très élevé et les qualités mécaniques médiocres.

Un autre but de l'invention est d'éviter de provoquer de problèmes de corrosion galvanique.

Un autre but de l'invention est de permettre l'utilisation d'une telle endoprothèse dans des vaisseaux de très faible diamètre, surtout dans des conditions limites de contraste, typiquement pour des opérations intra-cérébrales.

L'objet de l'invention est une endoprothèse munie d'une armature en fils métalliques, et typiquement un stent.

L'endoprothèse selon l'invention est caractérisée en ce que son armature est formée d'au moins trois couches de fils et les fils formant l'armature comprennent une âme en un matériau radio-opaque dit « absorbeur » ; ces moins trois couches de fils, vues latéralement, se présente comme un empilement de fenêtres superposées et décalées entre elles dans l'espace et offrent, en perspective, l'apparence d'une série de troncs de pyramide. L'interaction entre les âmes des fils des différentes couches améliore la visibilité de la dite endoprothèse en imagerie médicale sous rayons X.

Un avantage inattendu de cette conception est que l'on peut utiliser de très faibles quantités de matériau radio-opaque dit « absorbeur » tout en gardant une visibilité d'une qualité surprenante, comme on va le montrer ci-après.

Un autre but de l'invention est de limiter au maximum les mouvements relatifs des différentes couches de l'armature entres elles.

A cette fin, les fils formant les au moins trois couches sont de préférence entrelacés entre eux, de sorte que chaque fil fait partie de chacune des couches.

Suivant un mode de réalisation avantageux, le diamètre de l'âme des fils n'excède pas 25 µm, et de préférence n'excède pas 13 µm.

Le matériau radio-opaque de l'âme est choisi avantageusement au sein de l'ensemble suivant [or, platine, tantale].

Le métal entourant l'âme radio-opaque est choisi de préférence au sein de l'ensemble suivant [acier inoxydable, Elgiloy, alliages de nickel, alliages de titane].

Le métal entourant l'âme est avantageusement un matériau à mémoire de forme.

Suivant un mode de réalisation avantageux, les fils sont réalisés par co-extrusion.

Suivant un mode de réalisation particulier, l'armature est dépourvue de revêtement. Dans ce cas, l'endoprothèse forme ce que l'on appelle un « stent ».

Dans la description qui va suivre, on utilisera indifféremment le terme stent ou endoprothèse, sans que l'usage de l'un ou l'autre de ces termes entraîne une limitation quelconque à un mode de réalisation particulier, sauf indication explicite.

### Brève description des figures

Ces aspects ainsi que d'autres aspects de l'invention seront clarifiés dans la description détaillée de modes de réalisation particuliers de l'invention, référence étant faite aux dessins annexés, dans lesquelles :
La Fig.1 est une vue schématique du principe optique de l'imagerie
La Fig.2 est un tableau mettant en parallèle différents diamètres de fils constitués d'un matériau radiopaque et les « coefficients de visibilité » correspondants ;
La Fig.3 est une vue en gros plan d'un maillage de stent multi-couches ;

Les figures ne sont pas dessinées à l'échelle. Généralement, des éléments semblables sont dénotés par des références semblables dans les figures.

### Description détaillée de modes de réalisation particuliers

La radiopacité se traduit par l'atténuation des rayons X, lors de leur passage à travers la matière. Dans le cadre de l'invention, on ne parlera ici bien sûr des rayons X que pour des niveaux énergétiques compatibles avec une utilisation en imagerie médicale. La proportion de rayons X arrêtés par la matière traversée conditionne en effet le niveau de contraste de gris visible sur l'image.

L'intensité des rayons se réduit à mesure qu'ils traversent la matière parce que des photons peuvent être arrêtés dans la direction de propagation par un effet dit photoélectrique.

L'affaiblissement de l'intensité obéit à une loi exponentielle d'atténuation qui se définit sous la forme: Iₓ = I₀ e^{-µd}, où I₀ est l'intensité du faisceau de rayons X incident et Iₓ, l'intensité du faisceau absorbée à une distance d et p est le coefficient d'atténuation linéaire en cm⁻¹ (ceci implique qu'une partie de l'énergie seulement est absorbée). L'atténuation globale du faisceau de rayons X est responsable du noircissement (ou de la brillance) global(e) de l'image radiographique.

L'atténuation des rayons X dépend de l'épaisseur de l'objet ou du matériau, de sa densité, du numéro atomique des atomes du composant et de l'énergie des rayons X.

Il faut remarquer que plus les rayons sont énergétiques, plus l'atténuation est réduite, et les rayons pénétrants.

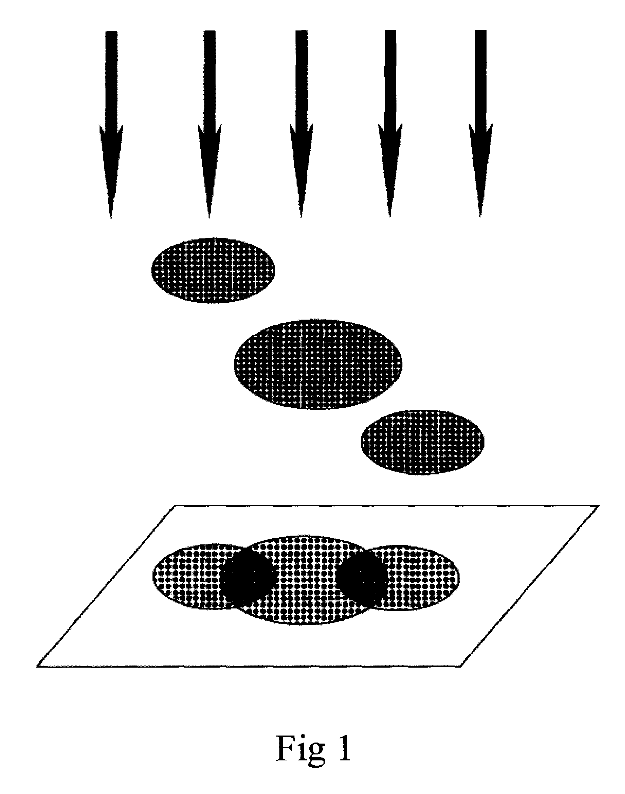

L'image radiographique sera donc formée par les différences d'atténuation ou d'absorption d'un faisceau de rayons X en fonction des milieux traversés. Le niveau de gris visible dans chaque zone du film correspond à la somme des atténuations élémentaires engendrée par les obstacles (os, masses musculaires, parois des vaisseaux, armature de stents) successivement traversés. Les « ombres » de ces obstacles apparaissent ainsi superposées les unes sur les autres sans qu'il soit possible de les différencier (Fig. 1).

### Principe de l'arrêt des rayons X

Un effet dit photoélectrique se produit lorsqu'un rayon X passe à proximité d'un électron d'une couche profonde d'un atome avec suffisamment d'énergie pour pouvoir l'éjecter.

Le rayon X est absorbé et le saut énergétique qui en résulte est transformé en un "photoélectron" d'énergie cinétique correspondante. Le "trou" laissé par l'électron éjecté est comblé par le saut d'un électron d'une couche plus extérieure, qui laisse échapper à son tour un rayon X₁ caractéristique de faible énergie en changeant d'orbite.

L'énergie du rayon X₁ peut être considérée ici comme négligeable, étant d'un niveau trop faible pour exercer un effet significatif sur l'image obtenue).

La probabilité d'interaction par un effet photoélectrique est proportionnelle à la densité du matériel et au cube du numéro atomique des atomes constituants. Les atomes de numéro atomique élevé, comme le platine (Z=78) ou le plomb (Z=82) arrêtent plus facilement les rayons X par un effet photoélectrique que les atomes de numéro atomique faible (carbone, hydrogène, oxygène, azote) composant la matière organique. Par exemple, la probabilité d'interaction par un effet photoélectrique est (78/16)³ = 11,5 fois plus grande pour un atome de platine (Z = 78) que pour un atome d'oxygène (Z = 16).

On considère que l'effet photoélectrique est l'effet prédominant pour des tensions entre 50 et 70 kV.

La logique voudrait donc que l'on utilise des stents comprenant d'importantes quantités de métaux de nombre atomique élevé.

Cette « logique » est cependant impossible à appliquer telle quelle en imagerie médicale. Il faut d'abord que ces matériaux soient médicalement compatibles avec un séjour indéterminé dans le corps humain, ce qui exclut d'emblée des matériaux tels que le plomb, l'uranium etc. Quant aux matériaux « nobles », or, platine, tantale, leur prix exclut la possibilité de les utiliser en grandes quantités et leur mise en oeuvre dans une armature est difficile .

Par ailleurs, si l'on souhaite travailler sur des vaisseaux extrêmement fins, le volume de l'endoprothèse elle-même commence à poser un problème. On est donc obligé d'utiliser une armature formée à partir d'une quantité de métal tellement réduite que l'image qui en résulte devient inutilisable, a fortiori lorsque le bruit de fond est important (présence l'os), situation typique lorsqu'on travaille dans le domaine cérébral.

Il ne faut par ailleurs pas oublier que cette armature, aussi réduite soit-elle, est destinée à assumer un rôle fondamental dans la fonction de l'endoprothèse : soutenir la paroi du vaisseau dans laquelle elle est insérée. En conséquence, il est nécessaire de recourir à des métaux présentant des qualités mécaniques élevées (élasticité, résistance à la flexion, etc.), ce qui ne va pas forcément de pair avec un nombre atomique élevé.

Beaucoup de chercheurs ont tenté d'associer un métal radio-opaque avec une âme en métal présentant de meilleures qualités mécaniques. Le placage (cladding) est, à cet égard, une des solutions les plus courantes, notamment car la quantité de métal « noble » utilisée peut être dosée de façon précise. Malheureusement, l'association de deux métaux baignant dans les fluides corporels donne lieu à l'apparition de réactions galvaniques, qui entraînent à terme la dissolution pure et simple d'un des métaux en contact.

Dans le cadre de l'invention, on a donc songé à utiliser, de préférence à des armatures tubulaires découpées, des armatures tressées, le métal radio-opaque servant de « marqueur » étant noyé dans les fils en matériau plus résistant, ce qui est possible par exemple en utilisant la technique de la co-extrusion.

Dans le cas qui nous préoccupe, il est apparu judicieux de coupler le fil métallique avec un absorbeur de rayon X puissant tel que le platine. Un tube en Nitinol est co-extrudé à 30% avec une âme en platine et ensuite étiré au diamètre souhaité. Ce fil est fournis par Fort Wayne Métal sous la dénomination Nitinol-DFT ® (Drawn Filled Nitinol Tube).

Si pour un fil Nitinol -DFT® de 40pm de diamètre est chargé de 30% de platine, le diamètre du corps en platine se situe autour de 22 µm. En pratique on estime, pour les absorbants les plus puissants tels que l'or ou le platine, que la valeur de 22 µm constitue la limite inférieure de la détection aux rayons X.

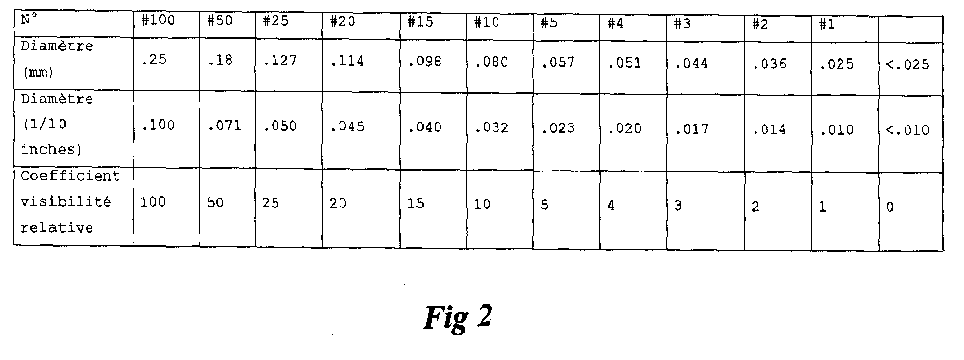

La Fig.2 est un tableau mettant en parallèle les coefficients de visibilité relatifs de fils de platine de diamètres croissants. Comme on peut le voir sur ce tableau, la limite inférieure est atteinte pour un diamètre de fils de 0.025 mm. Au-dessous de cette valeur, le signal généré se confond avec le bruit de fond de l'appareil d'imagerie médicale. Encore une fois, on se heurte au problème de la limite pratique de la détection.

Or les stents pour le traitement d'anévrismes cérébraux se caractérisent par leur faible diamètre, en général de l'ordre de 3-5 mm. Les fils du stent doivent donc être suffisamment fins (de l'ordre de 40 à 50 µm) pour pouvoir être introduits via un cathéter dont le diamètre extérieur ne dépasse pas 3 Fr (French).

Selon la loi d'atténuation mentionnée plus haut, l'épaisseur du matériau traversé est un facteur important : plus le matériau est épais plus l'absorption est importante. On a eu l'idée de recourir à une armature formée de plusieurs couches maillées successives de matériau, de façon à vérifier si une « accumulation » de couches maillées successives pouvait jouer le même rôle qu'une couche continue épaisse de matériau radiopaque.

Dans le cas d'une armature ou d'un stent tressé, cet effet est aisé à obtenir en utilisant une technique de tressage multi-couche, telle que décrite dans la demande (PCT/BE01/00210) du même inventeur.

Dans le cas qui nous préoccupe, il est apparu judicieux de coupler le fil métallique avec un absorbeur de rayon X puissant tel que le platine. Un fil de platine est inséré dans un tube en Nitinol (le fil représentant 30% de la section du tube), le tout est ensuite étiré au diamètre souhaité. Ce fil est fournis par Fort Wayne Métal sous la dénomination Nitinol-DFT ® (Drawn Filled Nitinol Tube).

Si un fil Nitinol -DFT ® de 40 µm de diamètre est chargé de 30% de platine, le diamètre de l'âme en platine se situe autour de 22 µm. Comme signalé plus haut, en pratique on estime, pour les absorbants les plus puissants tels que l'or ou le platine, que la valeur de 22 µm constitue la limite inférieure de la détection aux rayons X.

Comme on a à faire à un maillage et non à une plaque de métal continue, la quantité de platine mise en oeuvre est insignifiante. Pourtant, un phénomène inattendu fait son apparition. En dépit des faibles quantités de métal impliquées, la radiopacité globale du stent se trouve considérablement renforcée ou amplifiée. Ce phénomène peut s'expliquer comme suit :

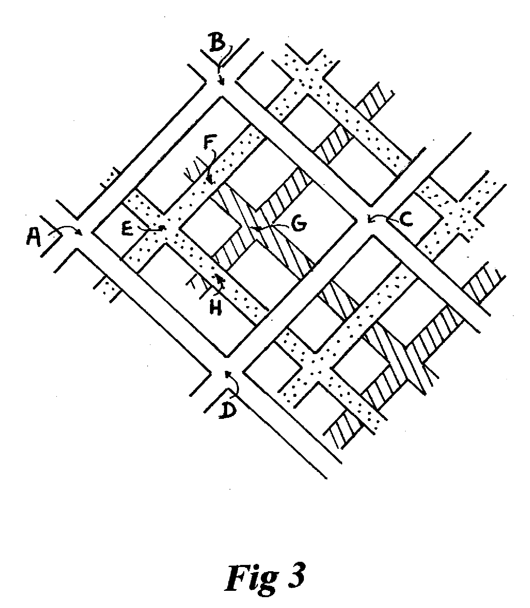

En se référant à la Fig. 3, on remarque qu'une armature à plusieurs couches -trois, en l'occurrence- vue latéralement se présente comme une superposition de fenêtres superposées dans l'espace. Par soucis de clarté, on a négligé de représenter les entrelacements ; les mailles de chaque couche apparaissent donc comme des quadrilatères évidés.

La couche la plus extérieure est représentée en blanc, la couche intermédiaire est pointillée, la couche la plus intérieure est hachurée.

Ces fenêtres étant décalées entre elles, elles offrent, en perspective, l'apparence d'une série de troncs de pyramide.

L'espace entre les fils d'une couche intérieure du stent (EFGH) apparaît en effet comme « plus petit » que l'espace entre les fils d'une couche extérieure (ABCD). Ce phénomène apparaît d'autant plus marqué que le nombre de couches s'accroît.Les mailles des quadrilatères élémentaires de chaque paroi se superposent les unes en dessous des autres jusqu'à l'infini, formant des cônes dont la base (ABCD) coïncide avec la couche la plus extérieure de l'armature.

Un flux de rayons X émis par la source utilisée par un appareil d'imagerie médicale rencontre la surface du stent sous un angle d'incidence donné. L'âme en platine de chaque fil va absorber environ 78% des rayons. La partie non absorbée, au lieu de se disperser ou d'être absorbée par l'os ou les tissus environnant, est absorbée de proche en proche par les fils voisins.

Cet arrangement successif de couches présentant des fils de même orientation offre l'avantage que les rayons X dispersés, les rayons X secondaires et les rayons non absorbés par l'os et les tissus sont retenus pour tous les angles d'incidences. Un stent d'une telle configuration joue donc le rôle d'une grille anti-dispersion.

La réalité de ce phénomène est étayée par les 2 expérimentations suivantes.

Exemple 1: Il est signalé plus haut que l'épaisseur limite inférieurs pour les métaux les plus absorbants est 22 µm. Il se trouve que le fournisseur dispose d'un fil DFT de 44 µm avec 10% de platine, ce qui donne une épaisseur de platine de 13 µm.

Un stent formé d'une tresse comme décrit plus haut réalisée avec ce fil produit lors des essais une image parfaitement visible et plus contrastée sur l'os et les tissus environnant que le stent témoin.

Exemple 2: Un stent à paroi simple, tressé avec un fil de 40 µm de diamètre contenant 30% de platine montre certes une radiopacité aux rayons X, mais dès qu'il est placé dans un environnement osseux, il n'est plus décelable visuellement.

Cet exemple démontre que l'os absorbe tous les rayons perdus et non absorbés dans les environs du stent, Ce qui accroît le bruit de fond dû à l'os. Un tel phénomène est absent dans le cas d'un stent à simple paroi.

### Effet sur les anévrismes

L'effet décrit plus haut peut être obtenu en plaçant l'un à l'intérieur l'autre plusieurs stents (ou endoprothèses) de diamètres correspondants, mais il s'agit là d'une méthode peu pratique, notamment du fait de la difficulté d'alignement de ces stents (ou endoprothèses) et d'un éventuel problème de migration (déplacement relatif de chacun des stents par rapport à la paroi d'un vaisseau sanguin). Les essais montrent que l'on obtient un bien meilleur résultat avec un stent unique tressé à plusieurs couches.

Un problème que l'on cherche à résoudre avec la pose d'endoprothèses ou de stents cérébraux est celui de prévenir le développement d'anévrismes, dont la rupture entraîne des effets fréquemment fatals pour le patient.

Les anévrismes cérébraux sont classés selon leur taille, qui prend en compte à la fois le diamètre du sac anévrismal mais aussi la largeur du collet (passage entre le sac anévrismal et le vaisseau sanguin sur lequel l'anévrisme s'est développé). Ils sont classifiés différemment selon les auteurs. Higashida, par exemple, classifie les anévrismes en 3 catégories, les anévrismes de taille « petite à moyenne » lorsque la poche reste inférieure à 12 mm , les anévrismes « larges » de 12 à 25 mm et les anévrismes « géants » supérieurs à 25 mm.

Traiter les anévrismes cérébraux se fait généralement par l'acheminement d'un «coil» placé dans la poche de l'anévrisme pour le colmater. L'état actuel de la technique montre que ce traitement peut être efficace pour les anévrismes à petit collet (2 à 3 mm), sachant que la taille de ces anévrismes peut varier, et surtout s'accroître.

La plupart des anévrismes présentent en fait un collet dont la taille dépasse 4 mm; le traitement par « coil» de ces anévrismes, dit géants, ne permet pas de les traiter efficacement : Dans bon nombre de cas, le praticien est amené à placer plusieurs « coils » sans que l'anévrisme thrombose avec succès. Un phénomène pernicieux peut de surcroît apparaître : la migration de ces coils vers l'artère principale. Pour éviter ce problème, certains praticiens placent au préalable dans l'artère, au droit du collet de l'anévrisme, un stent assumant la fonction d'un dispositif anti-retour, généralement un cylindre en Nitinol découpé au laser de façon à former de larges mailles. Ils insèrent ensuite un ou plusieurs coils dans l'anévrisme au travers des mailles du stent, ce dernier empêchant un retour ultérieur du coil vers l'artère. Ce procédé, en plusieurs étapes, se traduit par une procédure longue, fastidieuse et coûteuse, pour un taux d'échec élevé. Particularités du traitement par stent à double paroi

L'entrée du sang dans un anévrisme saculaire se fait par la partie supérieure du collet (distale par rapport à l'arrivée du flux) ; le flux entraîné crée un tourbillon (vortex) qui se déplace tout le long de la paroi saculaire jusqu'à sa sortie. Ce vortex est suivi par un autre, puis par un autre, qui, rythmés par les battements du coeur, se suivent d'une manière continue. Le fait que ces vortex ne suivent pas rigoureusement la même trajectoire dans l'anévrisme crée une perturbation (désordre tourbillonnant) qui maintient la pression contre la paroi du sac anévrismal, laquelle s'amincit peu à peu en se dilatant, et ce jusqu'à la rupture éventuelle.

On a cherché à modifier cette hémodynamique dans l'anévrisme en bloquant le mouvement de ces tourbillons, ce qui favorise la stagnation du sang et par conséquent la coagulation (thrombose) dans la poche même de l'anévrisme.

Différentes tentatives ont été faites pour créer cette thrombose en mettant une endoprothèse ou un stent classiques (cylindre découpé au laser, à dilatation par ballon ou tresse monocouche) à l'entrée de l'anévrisme.

Il s'est avéré que d'une part les stents ne produisaient pas l'effet positif escompté (dans le sens de l'induction d'une thrombose) sur le flux sanguin entrant. A l'analyse, au regard de la surface du collet, leur maillage représente une surface de recouvrement (projection des mailles sur le plan du collet) très faible. Le rapport [surface du collet/surface occupée par les mailles] est donc faible. Augmenter ce rapport poserait un problème d'encombrement dans le cathéter de pose, avec pour conséquence une difficulté accrue d'insertion et de navigation dans les artères cérébrales, qui sont par nature particulièrement tortueuses.

De leur côté, les endoprothèses constituées d'une armature rigide recouverte d'un film (en polyester ou en PTFE) posent des problèmes d'encombrement et de réactions inflammatoires liées aux molécules polymères.

Par contre, on constate que la configuration proposée par l'invention, soit généralement une tresse multicouche où les différentes couches sont superposées l'une à l'autre et décalées entre elles, confère au stent utilisé un rôle important dans l'altération du flux, qui finit par stagner, favorisant l'hémostase qui met essentiellement en jeu les plaquettes sanguines (thrombocytes). Cette constatation est a priori tout à fait paradoxale, puisque, les fils étant plus fins, le recouvrement [surface du collet/surface occupée par les mailles] est du même ordre que précédemment.

Afin de fermer l'anévrisme efficacement d'un côté et assurer la perméabilité des branches qui peuvent se trouver en face de l'anévrisme ou quelques millimètres en amont et en aval de ce dernier, on a utilisé un stent de porosité équivalente à celle d'un stent monocouche (c'est-à-dire 70 à 80%) mais en utilisant un arrangement tridimensionnel différent des pores et les couches afin d'obtenir une perméabilité plus réduite, suffisante pour atténuer la force que créent les vortex lorsqu'il se forment dès l'entrée dans l'anévrisme. La perméabilité à l'eau est définie comme la quantité d'eau (en gr/min.cm²) passant dans un appareil normalisé sous une pression correspondant à 120 mmHg). En l'occurrence, sans le stent elle est de 14260 gr/min.cm² (soit 100%) et avec un stent de 3mm est de l'ordre de 12162 ± 76 gr/min.cm², soit 85%.

La porosité est donc objectivement du même ordre de grandeur que celle d'un stent monocouche (par exemple du type d'un tube découpé au laser, ou tressé). Ce qui rend le stent multicouches plus efficace, est donc la manière dont les mailles sont réparties dans l'espace, autrement dit c'est la modification de la géométrie tridimensionnelle qui dicte l'efficacité du stent pour altérer l'hémodynamique et, en conséquence,thromboser l'anévrisme.

On notera un autre avantage que présente le stent de l'invention sur les endoprothèses recouvertes d'un film : il est fréquent que les anévrismes soient situés à proximité d'embranchements de vaisseaux collatéraux dans un vaisseau sanguin. Lorsque c'est le cas, le placement d'une endoprothèse recouverte risque d'obturer non seulement l'anévrisme, mais également ces embranchements de vaisseaux collatéraux. Un tel phénomène ne peut apparaître avec un stent non recouvert, au-travers duquel le flux de sang passe sans problème.

Par ailleurs, le tressage permet au stent d'adopter une section minimale lors de son introduction (sous diamètre réduit) dans un cathéter.

Un dernier effet, et non des moindres, a pu être constaté en vérifiant a posteriori par des évaluations in vivo l'évolution de la resténose (accumulation de plaquettes sanguine) sur les parois d'un vaisseau recouvert intérieurement par un stent ou une endoprothèse. Par un effet non encore expliqué à ce jour, mais qui pourrait être lié aux frottements réciproques des fils formant les différentes couches de l'armature, on constate une absence surprenante de resténose. La tresse multicouche aurait donc une fonction « auto-nettoyante » inattendue, ce qui ouvre de nouvelles perspectives d'application pour ce type de stent.

Il apparaîtra évident pour l'homme du métier que la présente invention n'est pas limités aux exemples illustrés et décrits ci-dessus. L'invention comprend chacune des caractéristiques nouvelles ainsi que leur combinaison. La présence de numéros de référence ne peut être considérée comme limitative. L'usage du terme « comprend » ne peut en aucune façon exclure la présence d'autres éléments autres que ceux mentionnés. L'usage de l'article défini « un » pour introduire un élément n'exclut pas la présence d'une pluralité de ces éléments. La présente invention a été décrite en relation avec des modes de réalisations spécifiques, qui ont une valeur purement illustrative et ne doivent pas être considérés comme limitatifs.

## Revendications

1. Endoprothèse munie d'une armature en fils métalliques **caractérisée en ce que**
- l'armature est formée d'au moins trois couches de fils
- les fils formant l'armature comprennent une âme en un matériau radio-opaque,
- ces moins trois couches de fils, vues latéralement, se présente comme un empilement de fenêtres superposées et décalées entre elles dans l'espace et offrent, en perspective, l'apparence d'une série de troncs de pyramide, l'interaction entre les âmes des fils des différentes couches améliorant la visibilité de la dite endoprothèse en imagerie médicale sous rayons X.

2. Endoprothèse selon la revendication 1, **caractérisée en ce que** les fils formant les au moins trois couches sont entrelacés entre eux, de sorte que chaque fil fait partie de chacune des couches.

3. Endoprothèse selon l'une quelconque des revendications 1 ou 2, **caractérisée en ce que** le diamètre de l'âme des fils n'excède pas 25 µm.

4. Endoprothèse selon l'une quelconque des revendications 1 à 3, **caractérisée en ce que** le diamètre de l'âme n'excède pas 21 µm.

5. Endoprothèse selon l'une quelconque des revendications 1 à 4, **caractérisée en ce que** le diamètre de l'âme n'excède pas 13 µm.

6. Endoprothèse selon l'une quelconque des revendications 1 à 5, **caractérisée en ce que** le matériau radio-opaque de l'âme est choisi au sein de l'ensemble suivant [or, platine, tantale].

7. Endoprothèse selon l'une quelconque des revendications 1 à 6, **caractérisée en ce que** le métal entourant l'âme radio-opaque est choisi au sein de l'ensemble suivant [acier inoxydable, Elgiloy, alliages de nickel, alliages de titane].

8. Endoprothèse selon la revendication 7, **caractérisée en ce que** le métal entourant l'âme est un matériau à mémoire de forme.

9. Endoprothèse selon l'une quelconque des revendications 1 à 8, **caractérisée en ce que** les fils sont réalisés par co-extrusion.

10. Endoprothèse selon l'une quelconque des revendications 1 à 8, **caractérisée en ce que** l'armature est dépourvue de revêtement.
